# EUROPÄISCHE PATENTSCHRIFT

(11) **EP 3 674 261 B1**
(45) Veröffentlichungstag und Bekanntmachung des Hinweises auf die Patenterteilung: **27.10.2021**
(21) Anmeldenummer: 18248041.8
(22) Anmeldetag: 27.12.2018
(51) Int. Cl.: C01B 3/38, C01B 3/50, B01D 53/14, B01D 53/22, C07C 29/152

(54) **VERFAHREN ZUR SYNTHESE EINER WASSERSTOFFHALTIGEN VERBINDUNG**
METHOD FOR THE SYNTHESIS OF A HYDROGEN-CONTAINING COMPOUND
PROCÉDÉ DE SYNTHÈSE D'UN COMPOSÉ CONTENANT DE L'HYDROGÈNE

(43) Veröffentlichungstag der Anmeldung: 01.07.2020
(73) Patentinhaber: GasConTec GmbH, 61348 Bad Homburg v. d. Höhe (DE)
(72) Erfinder: Koss, Ulrich, 61348 Bad Homburg (DE); Rappold, Dorit, 60437 Frankfurt ( Niedereschbach) (DE)
(74) Vertreter: Patentanwälte Bauer Vorberg Kayser

(56) Entgegenhaltungen:
- EP-A1- 0 047 596
- EP-A1- 3 284 733
- EP-A2- 0 233 076
- WO-A1-2017/137581
- US-A- 4 181 675
- US-A- 4 348 486

## Beschreibung

Die Erfindung betrifft ein Verfahren zur Synthese von Methanol gemäß dem Oberbegriff von Anspruch 1 sowie eine Anlage zur Synthese von Methanol gemäß dem Oberbegriff von Anspruch 15.

Bei der Methanolherstellung aus einem Rohstoff wie etwa Erdgas fällt Kohlenstoffdioxid als Abgas an. In der Regel wird dieses Kohlenstoffdioxid als Bestandteil eines Verbrennungsabgases bei niedrigem Druck in die Atmosphäre abgegeben. Das Kohlenstoffdioxid macht dabei lediglich einen Anteil zwischen 5 % und 30 % des Verbrennungsabgases aus. Das Verbrennungsabgas kann dabei in dem Ofen eines Dampfreformers oder in einer befeuerten Heizvorrichtung zum Erhitzen eines Prozessstroms entstehen. Diese Vorrichtungen werden sowohl mit dem Erdgas als auch mit sonstigen Restgasen befeuert, die an verschiedenen Stellen einer Methanolanlage anfallen. Regelmäßig werden etwa 50 % bis 80 % der Kohlenstoffatome in dem Rohstoff zum Bestandteil des erzeugten Methanols, sodass die restlichen Kohlenstoffatome in dem Rohstoff - also bis zu 50 % - im Wesentlichen zu Kohlenstoffdioxid in dem Verbrennungsabgas werden.

In die Atmosphäre abgegebenes Kohlenstoffdioxid stellt ein Risiko für das Weltklima dar. Insbesondere daher gibt es auch weltweit zunehmend Gesetzgebung, welche die großvolumige Abgabe von Kohlenstoffdioxid in die Umgebung einschränkt oder verbietet.

Das Extrahieren und Speichern von Kohlenstoffdioxid aus dem Verbrennungsabgas eines Ofens oder einer befeuerten Heizvorrichtung, beispielsweise durchgeführt mit einer Ammoniakwäsche, einer Aminwäsche, oder durch ein anderes absorptives Waschverfahren, ist aus dem Stand der Technik bekannt. Beispielhaft zu nennen sind die Offenlegungsschriften EP 2230000 A1, EP2564915 A1 und EP2678093 A1.

Diese Ansätze aus dem Stand der Technik sind aber so energetisch aufwändig und teuer, dass sie den Wirkungsgrad der Anlage deutlich absenken und die Investitionskosten deutlich ansteigen lassen. Ferner sind die dafür erforderlichen Vorrichtungen sehr groß und die mit den Wäschen behandelten Abgase können Spuren der Absorptionsmittel oder von Reaktions- oder Zersetzungsprodukten der Absorptionsmittel enthalten, die ihrerseits eine potentielle Gesundheits- oder Umweltbelastung darstellen können.

Beispielsweise aus der US2014080071 A1 ist eine Möglichkeit bekannt, welche aber ebenfalls energetisch aufwändig und teuer ist, die befeuerte Heizvorrichtung auf die sogenannte OxyFuel -Technologie umzustellen. Dies geschieht, indem die zugeführte Verbrennungsluft durch ein Gemisch ersetzt wird, welches aus in einer Luftzerlegungseinrichtung erzeugtem Sauerstoff und rückgeführtem CO2 besteht. Das Verbrennungsabgas aus der in der Heizvorrichtung durchgeführten Verbrennung besteht dann überwiegend nur noch aus Wasserdampf und CO2.

Aus der EP 3 284 733 A1, von welcher die Erfindung als nächstkommend ausgeht, ist ein Verfahren und eine Anlage zur Synthese von Methanol bekannt, bei der Kohlenstoffdioxid aus einem Gasstrom, welcher als Restgas einer einem Reaktor nachgelagerten Methanolkondensation erhalten wird, mittels Ammoniak ausgewaschen wird. Die Wäsche mittels Ammoniak ermöglicht es dabei, das ausgewaschene Kohlenstoffdioxid einerseits mit einem hohen Reinheitsgrad und andererseits mit einem hinreichend hohen Druck zu erhalten, sodass es mit weniger Aufwand gespeichert werden kann.

Es bleibt jedoch bei der Kohlenstoffdioxidbelastung der Atmosphäre durch andere kohlenstoffdioxidhaltige Emissionsquellen der Methanolanlage, wie insbesondere Verbrennungsabgase, z. B. aus der befeuerten Heizvorrichtung der Methanolanlage. Diese Heizvorrichtung wird in der Regel mit Erdgas und/oder anderen kohlenstoffhaltigen Restgasströmen aus der Methanolanlage befeuert. Diese Verbrennungsabgase stellen in der Regel einen beträchtlichen Anteil, nämlich zwischen 30 % und 70%, der Kohlenstoffdioxidemission der Methanolproduktionsanlage dar. Sie können mit der aus der EP 3 284 733 A1 bekannten Methode nicht vermieden werden.

Ausgehend von diesem Stand der Technik besteht die Aufgabe der Erfindung daher darin, das aus dem Stand der Technik bekannte Verfahren und die aus dem Stand der Technik bekannte Anlage dahingehend zu verbessern, dass auch die Kohlenstoffdioxidemission durch andere Emissionsquellen der Methanolanlage wie insbesondere durch die Verbrennungsabgase verringert werden kann und somit ein Großteil der Kohlenstoffdioxidbelastung der Methanolproduktionsanlage insgesamt vermieden werden kann.

Bezogen auf ein Verfahren von Methanol gemäß dem Oberbegriff von Anspruch 1 wird diese Aufgabe durch die Merkmale des kennzeichnenden Teils von Anspruch 1 gelöst. Bezogen auf eine Anlage zur Synthese von Methanol gemäß dem Oberbegriff von Anspruch 15 wird diese Aufgabe durch die Merkmale des kennzeichnenden Teils von Anspruch 15 gelöst.

Der Erfindung liegt die Erkenntnis zugrunde, dass die Kohlenstoffdioxidbelastung der Umwelt weiter dadurch verringert werden kann, dass befeuerte Heizvorrichtungen und ähnliche Apparate durch ein Gas gespeist werden können, welches zu einem großen Teil aus Wasserstoff besteht, da die Verbrennung des Wasserstoffes ja nur zu Wasser führt. Für diese Verringerung ist jedoch kein besonders reiner Wasserstoffstrom erforderlich, vielmehr ist das Vorhandensein weiterer Bestandteile wenig schädlich. Daher ist für die Bereitstellung eines solchen Gases auch keine Druckwechsel-Adsorptionsanlage erforderlich, welche einen Wasserstoffstrom sehr hoher Reinheit bereitstellen kann. Vielmehr kann beispielsweise eine Membranvorrichtung zur Abtrennung des Wasserstoffs verwendet werden. Solche Alternativen bieten nämlich zwar eine geringere Reinheit des Wasserstoffstroms, stellen aber auch das Restgas mit einem höheren Druck bereit, sodass dieses Restgas auch ohne nachfolgende Komprimierung oder jedenfalls mit geringerer Komprimierung etwa zum Reaktor für die Synthesegasherstellung zurückgeführt werden kann. Auf diese Weise kann also sowohl die Umweltbelastung verringert als auch der Energiebedarf für den Betrieb der Anlage verringert werden. Im Ergebnis kann ein großer Teil, beginnend mit etwa 30 %, der Emissionen von Kohlenstoffdioxid in die Atmosphäre vermieden werden. Es kann sogar sein, dass Kohlenstoffdioxidemissionen in die Atmosphäre im Wesentlichen vollständig und damit im Wesentlichen zu 100 % vermieden werden können.

Weiter ist es auf dieser Grundlage möglich, einen Kohlenstoffdioxid-Produktstrom mit hoher Reinheit und mit einem hohen Druck zu erhalten. Die hohe Reinheit und der hohe Druck sind für die weitere Verarbeitung oder Speicherung des Kohlenstoffdioxids, etwa im Rahmen einer auch als CCS bezeichneten CO2-Sequestrierung, sehr vorteilhaft.

Die bevorzugte Variante des Unteranspruchs 3 sieht die Herstellung des Synthesegases durch eine autotherme Reformierung vor. Dies erlaubt es, auch das Synthesegas von vornherein mit einem hohen Druck bereitzustellen sodass auch an der Synthesegasherstellung nachgelagerten Stellen Prozessgas und daraus erhaltene andere Gase mit höheren Drücken bereitstehen.

Die bevorzugte Ausgestaltung des Unteranspruchs 7 beschreibt vorteilhafte Arten der dem Methanolreaktor nachgelagerten Abtrennung eines Restgasstroms von dem Rohmethanol, wohingegen die Unteransprüche 8 und 9 die Verwendung einer Membranvorrichtung als Wasserstofftrenner und diesbezügliche Merkmale genauer beschreiben.

Die Unteransprüche 10 bis 14 wiederum beschreiben vorteilhafte Ausgestaltungen des CO2-Entferners zum Gewinnen des Kohlenstoffdioxid-Produktstroms und insbesondere seiner Waschanordnung und Kompressoranordnung als mögliche Bestandteile.

Weitere Einzelheiten, Merkmale, Ziele und Vorteile der vorliegenden Erfindung werden nachfolgend anhand der nur Ausführungsbeispiele wiedergebenden Zeichnung erläutert. In der Zeichnung zeigt
- Fig. 1: schematisch das Fließbild einer Anlage zur Ausführung des vorschlagsgemäßen Verfahrens gemäß einem Ausführungsbeispiel,
- Fig. 2: schematisch das Fließbild der Waschanordnung des CO2-Entferners der Anlage der Fig. 1,
- Fig. 3: schematisch das Fließbild der Kompressoranordnung des CO2-Entferners der Anlage der Fig. 1 und

Das vorschlagsgemäße Verfahren dient der Synthese von Methanol. Es kann auch ein Stoff erhalten werden, welcher aus einer Weiterverarbeitung von Methanol erhalten wird. Nachstehend wird das vorschlagsgemäße Verfahren anhand der in der Zeichnung gezeigten vorschlagsgemäßen Anlage erläutert.

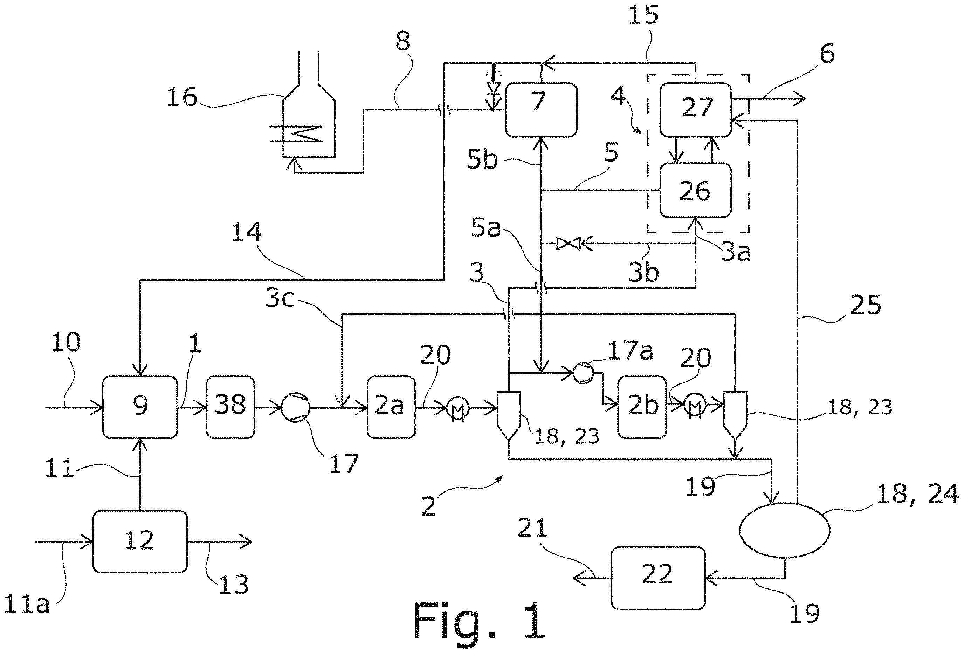

Gemäß dem vorschlagsgemäßen Verfahren wird ein Synthesegasstrom 1 mit Wasserstoff und Kohlenstoffoxiden einer Methanol-Reaktoranordnung 2 zur teilweisen Umwandlung in Methanol zugeführt. Neben Wasserstoff und Kohlenstoffoxiden kann der Synthesegasstrom noch weitere Bestandteile wie Stickstoff, Methan oder Edelgase aufweisen. Die teilweise Umwandlung des Synthesegasstroms 1 in Methanol erfolgt dabei in an sich aus dem Stand der Technik bekannter Weise. Die Methanol-Reaktoranordnung 2 kann grundsätzlich eine beliebige Anzahl von Reaktorstufen 2a aufweisen, beispielsweise auch nur eine Reaktorstufe 2a. In dem Ausführungsbeispiel der Fig. 1 weist die Methanol-Reaktoranordnung 2 zwei prozesstechnisch seriell angeordnete Reaktorstufen 2a, b auf.

Vorschlagsgemäß wird aus der Methanol-Reaktoranordnung 2 ein Methanol-Restgasstrom 3 erhalten, welcher Methanol-Restgasstrom 3 zumindest teilweise einem CO2-Entferner 4 zugeführt wird, aus welchem CO2-Entferner 4 ein Synthese-Recyclestrom 5 und ein CO2-Produktstrom 6 erhalten wird. Der Methanol-Restgasstrom 3 besteht bevorzugt überwiegend aus unreagiertem Synthesegas aus der Methanol-Reaktoranordnung 2.

Vorschlagsgemäß weist der CO2-Produktstrom 6 einen höheren molaren Kohlenstoffdioxidanteil auf als der Methanol-Restgasstrom 3. Insbesondere kann der CO2-Produktstrom 6 im Wesentlichen aus Kohlenstoffdioxid bestehen. Ebenso ist entsprechend bevorzugt, dass der CO2-Produktstrom 6 einen höheren molaren Kohlenstoffdioxidanteil aufweist als der Synthese-Recyclestrom 5.

Vorschlagsgemäß wird - wie aus der Fig. 1 ersichtlich - der Synthese-Recyclestrom 5 teilweise der Methanol-Reaktoranordnung 2 zugeführt. Das vorschlagsgemäße Verfahren ist dabei dadurch gekennzeichnet, dass der Synthese-Recyclestrom 5 teilweise einem Wasserstofftrenner 7 zugeführt wird, aus welchem ein Trennstrom 8 erhalten wird, welcher einen höheren molaren Wasserstoffanteil aufweist als der Synthese-Recyclestrom 5. Der Teil des Synthese-Recyclestroms 5, welcher der Methanol-Reaktoranordnung 2 zugeführt wird, kann auch als erster Recycle-Teilstrom 5a bezeichnet werden. Entsprechend kann der Teil des Synthesegasstroms 5, welcher dem Wasserstofftrenner 7 zugeführt wird, als zweiter Recycle-Teilstrom 5b bezeichnet werden.

Grundsätzlich kann der Methanol-Restgasstrom 3 vollständig dem CO2-Entferner 4 zugeführt werden. Bevorzugt ist jedoch, dass gemäß der Darstellung der Fig. 1 ein Teil des Methanol-Restgasstroms 3 der Methanol-Reaktoranordnung 2 zugeführt wird, was also einer Rückführung zu der Methanol-Reaktoranordnung 2 entspricht. Diese Rückführung kann dadurch erfolgen, dass der Methanol-Restgasstrom 3 zwei Teilströme 3a, b aufweist, von denen der erste Teilstrom 3a dem CO2-Entferner 4 zugeführt. Der zweite Teilstrom 3b wiederum kann dann entweder dem Synthesegasstrom 1 zugeführt werden, und zwar wahlweise vor oder nach dem unten noch zu beschreibenden Synthesegaskompressor 17. Alternativ und wie in der Zeichnung gezeigt kann der zweite Teilstrom 3b auch demjenigen Teil des Synthese-Recyclestroms 5 zugeführt werden, welcher der Methanol-Reaktoranordnung 2 zugeführt wird. Im vorliegenden Ausführungsbeispiel handelt es sich dabei um den ersten Recycle-Teilstrom 5a.

Entsprechend dem vorschlagsgemäßen Verfahren dient die vorschlagsgemäße Anlage zur Synthese von Methanol. Die vorschlagsgemäße Anlage weist die Methanol-Reaktoranordnung 2 auf, welcher Methanol-Reaktoranordnung 2 der Synthesegasstrom 1 mit Wasserstoff und Kohlenstoffoxiden zur teilweisen Umwandlung in Methanol und zum Erhalten des Methanol-Restgasstroms 3 zugeführt wird. Weiter weist die vorschlagsgemäße Anlage den CO2-Entferner 4 auf, welchem der Methanol-Restgasstrom 3 zumindest teilweise zum Erhalten des Synthese-Recyclestroms 5 und des CO2-Produktstroms 6 zugeführt wird, wobei der CO2-Produktstrom 6 einen höheren molaren Kohlenstoffdioxidanteil aufweist als der Methanol-Restgasstrom 3 und wobei der Synthese-Recyclestrom 5 teilweise der Methanol-Reaktoranordnung 2 zugeführt wird.

Die vorschlagsgemäße Anlage ist dadurch gekennzeichnet, dass die Anlage den Wasserstofftrenner 7 aufweist, welchem zum Erhalten des Trennstroms 8 der Synthese-Recyclestrom 5 teilweise zugeführt wird und ferner dadurch, dass der Trennstrom 8 einen höheren molaren Wasserstoffanteil aufweist als der Synthese-Recyclestrom 5.

Grundsätzlich kann der Synthesegasstrom 1 auf beliebige Art und Weise gewonnen werden. Bevorzugt ist jedoch, dass der Synthesegasstrom 1 in einer Synthesegasreaktoranordnung 9 aus einem kohlenstoffhaltigen Energieträgerstrom 10 gewonnen wird. Speziell kann es sein, dass der kohlenstoffhaltige Energieträgerstrom 10 Erdgas umfasst oder im Wesentlichen aus Erdgas besteht. Wie in der Fig. 1 gezeigt und bevorzugt wird der Synthesegasreaktoranordnung 9 zum Gewinnen des Synthesegasstroms 1 ein sauerstoffhaltiger Strom 11 zugeführt. Bei diesem sauerstoffhaltigen Strom 11 kann es sich gemäß einer Variante um Umgebungsluft 11a handeln.

Prinzipiell kann der Synthesegasstrom 1 in der Synthesegasreaktoranordnung 9 auf beliebige Art und Weise gewonnen werden, so etwa durch Dampfreformierung. Bevorzugt und dem Ausführungsbeispiel der Fig. 1 entsprechend ist jedoch vorgesehen, dass in der Synthesegasreaktoranordnung 9 der Synthesegasstrom 1 durch eine autotherme Reformierung aus dem kohlenstoffhaltigen Energieträgerstrom 10 gewonnen wird. Speziell dabei ist dann bevorzugt, dass der sauerstoffhaltige Strom 11 aus einer Luftzerlegungsvorrichtung 12 zum Gewinnen eines Stickstoffstroms 13 gewonnen wird. Sowohl der Stickstoffstrom 13 als auch der sauerstoffhaltige Strom 11 können dann aus der Umgebungsluft 11a gewonnen werden. Dann kann es auch sein, dass der sauerstoffhaltige Strom 11 im Wesentlichen aus Sauerstoff besteht. Bei der an sich aus dem Stand der Technik bekannten autothermen Reformierung stellt eine katalytische partielle Oxidation die für die endothermen Reformierungsreaktionen erforderliche Wärme bereit. Die Synthesegasreaktoranordnung 9 kann auch einen Pre-Reformer oder eine Entschwefelung zur Vorbehandlung des kohlenstoffhaltigen Energieträgerstroms 10 aufweisen.

Bezüglich des Wasserstofftrenners 7 kann es sein, dass aus dem Wasserstofftrenner 7 neben den Trennstrom 8 auch weitere Ströme erhalten werden. So ist bevorzugt vorgesehen, dass aus dem Wasserstofftrenner 7 ein Reform-Recyclestrom 14 erhalten wird, welcher einen höheren molaren Methananteil aufweist als der Synthese-Recyclestrom 5. Dieser Methananteil rührt aus dem Methan, welches in dem Methanol-Restgasstrom 3 enthalten ist. Entsprechend weist der Reform-Recyclestrom 14 bevorzugt auch einen höheren molaren Methananteil auf als der Trennstrom 8. Bei dem Reform-Recyclestrom 14 handelt es sich vorzugsweise um den Rest des Synthese-Recyclestroms 5, welcher nach Abtrennung des Trennstroms 8 durch den Wasserstofftrenner 7 verbleibt.

Grundsätzlich kann dieser Reform-Recyclestrom 14 auf beliebige Art und Weise verwendet werden. Bevorzugt ist dabei, dass - wie in der Fig. 1 dargestellt - der Reform-Recyclestrom 14 der Synthesegasreaktoranordnung 9 zum Gewinnen des Synthesegasstroms 1 zugeführt wird. Das in dem Reform-Recyclestrom 14 enthaltene Methan kann dann in Synthesegas umgewandelt und damit für die Methanolsynthese genutzt werden. Ebenso kann der Trennstrom 8 grundsätzlich beliebig verwendet werden. Vorzugsweise wird jedoch der Trennstrom 8 einer befeuerten Heizvorrichtung 16 zur Verbrennung zugeführt. Die befeuerte Heizvorrichtung 16 kann etwa dazu eingerichtet sein, einen oder mehrere Prozessströme und/oder Prozessdampf zu erhitzen. Durch den erhöhten Wasserstoffanteil des Trennstroms 8 erzeugt die befeuerte Heizvorrichtung 16 entsprechend wenig Kohlenstoffdioxid.

Eine solche Erzeugung und Rückführung eines methanhaltigen Stroms wie des Reform-Recyclestroms 14 muss sich aber nicht auf den Wasserstofftrenner 7 beschränken. So ist es entsprechend der Darstellung in der Fig. 1 auch bevorzugt, dass aus dem CO2-Entferner 4 ein weiterer Reform-Recyclestrom 15 erhalten wird. Grundsätzlich kann auch der weitere Reform-Recyclestrom 15 auf beliebige Art und Weise verwendet werden. Vorzugsweise und wie in der Zeichnung dargestellt wird der weitere Reform-Recyclestrom 15 mit dem Reform-Recyclestrom 14 zusammengeführt. Daher wird auch vorzugsweise der weitere Reform-Recyclestrom 15 der Synthesegasreaktoranordnung 9 zum Gewinnen des Synthesegasstroms 1 zugeführt. Bevorzugt ist weiter, dass der weitere Reform-Recyclestrom 15 Methan aufweist. Es kann sich dabei um Methan handeln, welches im Methanol-Restgasstrom 3 enthalten war und nicht in den CO2-Produktstrom 6 aufgenommen wurde. Entsprechend kann der weitere Reform-Recyclestrom 15 einen höheren molaren Methananteil aufweisen als der Methanol-Restgasstrom 3.

Auch wenn bei der autothermen Reformierung der Synthesegasstrom 1 mit einem hohen Druck durch die Synthesegasreaktoranordnung 9 bereitgestellt werden kann, so kann eine weitere Druckerhöhung des Synthesegasstroms 1 für die Methanolsynthese vorteilhaft sein. Daher ist es bevorzugt, dass der Synthesegasstrom 1 vor Zuführung zu der Methanol-Reaktoranordnung 2 durch einen Synthesegaskompressor 17 auf einen Synthesedruck gebracht wird. Um eine kleinere Dimensionierung des Synthesegaskompressors 17 zu ermöglichen, kann es sein, dass der Synthese-Recyclestrom 5 dem Synthesegaskompressor 17 prozesstechnisch nachgelagert der Methanol-Reaktoranordnung 2 teilweise zugeführt wird. Diese Feststellung hinsichtlich der Zuführung zum Synthesegasstrom 1 bezieht sich auf denjenigen Teil des Synthese-Recyclestroms 5, welcher der Methanol-Reaktoranordnung 2 zugeführt wird, im vorliegenden Beispiel also auf den ersten Recycle-Teilstrom 5a. Auf diese Weise muss der Synthesegaskompressor 17 nicht auch noch zur Druckerhöhung des Synthese-Recyclestroms 5 ausgelegt werden.

Diese dem Synthesegaskompressor 17 prozesstechnisch nachgelagerte teilweise Zuführung des Synthese-Recyclestroms 5 kann einerseits prozesstechnisch der ersten Reaktorstufe 2a der Methanol-Reaktoranordnung 2 vorgelagert erfolgen. Diese Zuführung kann aber ebenso - wie in der Fig. 1 dargestellt - zwischen mehreren Reaktorstufen 2a, b der Methanol-Reaktoranordnung 2 erfolgen. In dem Fall, dass die Methanol-Reaktoranordnung 2 einen Zwischenkompressor 17a zwischen den Reaktorstufen 2a, b aufweist - wie in der Fig. 1 dargestellt - kann die teilweise Zuführung des Synthese-Recyclestroms 5 dem Zwischenkompressor 17a prozesstechnisch vorgelagert erfolgen.

Der Synthesegasstrom 1 kann grundsätzlich noch weitere Behandlungsschritte erfahren. Eine bevorzugte Variante sieht dabei vor, dass der Synthesegasstrom 1 vor Zuführung zu der Methanol-Reaktoranordnung 2 zumindest teilweise einer Shiftkonvertierung 38 für eine Wassergas-Shift-Reaktion zugeführt wird, und zwar vorzugsweise sodass ein molarer Anteil an Wasserstoff im Synthesegasstrom 1 erhöht wird. Dies bietet sich insbesondere dann an, wenn mehr wasserstoffreiches Gas im Trennstrom 8 zum Betreiben der befeuerten Heizeinrichtung 16 benötigt wird. Bevorzugt ist, dass der Synthesegasstrom 1 dem Synthesegaskompressor 17 prozesstechnisch vorgelagert der Shiftkonvertierung 38 zugeführt wird.

Die obige Erhöhung des molaren Anteils an Wasserstoff im Synthesegasstrom 1 erfolgt vorzugsweise so, dass ein der Shiftkonvertierung 38 zugeführter Teil des Synthesegasstrom 1 wieder zurückgeführt wird. Es kann aber auch sein, dass ein der Shiftkonvertierung 38 zugeführter Teil des Synthesegasstroms 1 einem hier nicht gezeigten weiteren CO2-Entferner zugeführt wird und ein aus dem weiteren CO2-Entferner erhaltener weiterer Trennstrom, welcher vorzugsweise wasserstoffhaltig ist, der befeuerten Heizvorrichtung 16 zur Verbrennung zugeführt wird. Ebenso kann aus dem weiteren CO2-Entferner ein weiterer CO2-Produktstrom erhalten werden, welcher vorzugsweise einen höheren molaren Kohlenstoffdioxidanteil aufweist als der Synthesegasstrom 1. Der weitere CO2-Entferner kann eine chemische Wäsche und/oder eine physikalische Wäsche zum Erhalten des weiteren Trennstroms und des weiteren CO2-Produktstroms aufweisen. Vorzugsweise weist der CO2-Entferner eine weitere Membranvorrichtung zum Abtrennen von Wasserstoff auf. Bevorzugt ist, dass der weitere Trennstrom aus einer Niederdruckseite der weiteren Membranvorrichtung erhalten wird. Entsprechend ist es auch bevorzugt, dass der weitere CO2-Produktstrom aus einer Hochdruckseite der weiteren Membranvorrichtung erhalten wird.

Grundsätzlich kann der Methanol-Restgasstrom 3 auf beliebige Art und Weise aus der Methanol-Reaktoranordnung 2 erhalten werden. Bevorzugt ist jedoch, dass die Methanol-Reaktoranordnung 2 eine Methanol-Trennvorrichtung 18 zum Gewinnen des Methanol-Restgasstroms 3 und eines Rohmethanolstroms 19 aus einem Reaktor-Produktstrom 20 umfasst. Der Rohmethanolstrom 19 wird dann vorzugsweise zum Gewinnen von Methanol 21 einer Destillation 22 zugeführt. Diese Methanol-Trennvorrichtung 18 kann auch - wie in der Fig. 1 gezeigt aus mehreren separaten Vorrichtungen bestehen.

Speziell kann es sein, dass die Methanol-Trennvorrichtung 18 eine Kondensationsvorrichtung 23 zum Gewinnen des Rohmethanolstroms 19 und des Methanol-Restgasstroms 3 aus dem Reaktor-Produktstrom 20 durch Kondensation umfasst. Speziell für den Fall, dass die Methanol-Reaktoranordnung 2 mehrere Reaktorstufen 2a, b aufweist, wie in der Fig. 1 gezeigt, kann die Methanol-Trennvorrichtung 18 auch mehrere solche Kondensationsvorrichtungen 23 umfassen. Wie in der Fig. 1 dargestellt kann es auch sein, dass ein weiterer Methanol-Restgasstrom 3c aus der Methanol-Trennvorrichtung 18 und insbesondere aus einer Kondensationsvorrichtung 23 der Methanol-Trennvorrichtung 18 gewonnen wird. Vorzugsweise wird dieser weitere Methanol-Restgasstrom 3c zur Methanol-Reaktorvorrichtung 2 zurückgeführt. Dies kann, wie in der Fig. 1 dargestellt, etwa dadurch erfolgen, dass der weitere Methanol-Restgasstrom 3c insbesondere dem Synthesegaskompressor 17 nachgelagert dem Synthesegasstrom 1 zugeführt wird.

Alternativ oder zusätzlich zu der Kondensationsvorrichtung 23 kann die Methanol-Trennvorrichtung 18 einen Entspannungs-Tank 24 zum Gewinnen eines Entspannungs-Restgasstroms 25 aus dem Reaktor-Produktstrom 20 und/oder aus dem Rohmethanolstrom 19 umfassen. In diesem Entspannungs-Tank 24 wird der Entspannungs-Restgasstrom 25 durch Entspannung des jeweils zugeführten Stroms erhalten. Ebenso wird der nunmehr entspannte Rohmethanolstrom 19 aus dem Entspannungs-Tank 24 erhalten. Gemäß der Darstellung der Fig. 1 kann auch der Entspannungs-Restgasstrom 25 dem CO2-Entferner 4 zugeführt werden. Vor allem wenn der aus der Kondensationsvorrichtung 23 gewonnene Rohmethanolstrom 19 dem Entspannungs-Tank 24 zugeführt wird, wird ein Entspannungs-Restgasstrom 25 erhalten, welcher im Wesentlichen aus Kohlenstoffdioxid besteht und daher bereits eine hohe Reinheit an Kohlenstoffdioxid aufweist. Daher kann - wie untenstehend noch beschrieben wird - eine ansonsten vorgesehene Wäsche des Entspannungs-Restgasstroms 25 etwa mit Methanol als Waschmedium entfallen.

Grundsätzlich kann der Wasserstofftrenner 7 nach einem beliebigen Prinzip zum zumindest teilweisen Abtrennen von Wasserstoff aus dem Synthese-Recyclestrom 5 funktionieren. Bezüglich der Funktionsweise des Wasserstofftrenners 7 ist jedoch speziell bevorzugt, dass der Wasserstofftrenner 7 eine Membranvorrichtung zum Abtrennen von Wasserstoff aufweist. Dies ermöglicht es, dass nach dem Abtrennen des Wasserstoffs verbliebene Gas - also den Reform-Recyclestrom 14 - bei vergleichsweise hohem Druck zu erhalten. Bevorzugt ist, dass der Trennstrom 8 aus einer Niederdruckseite der Membranvorrichtung und der Reform-Recyclestrom 14 aus einer Hochdruckseite der Membranvorrichtung erhalten wird. Das bedeutet insbesondere, dass der Trennstrom 8 mit einem niedrigeren Druck als der Reform-Recyclestrom 14 aus der Membranvorrichtung erhalten wird. Vorzugsweise wird ferner ein Teil des Reform-Recyclestroms 14 zum Trennstrom 8 ausgekreist. Insbesondere in denjenigen Fällen, in denen der Reform-Recyclestrom 14 in den Kreislauf der Methanolsynthese zurückgeführt wird, kann auf diese Weise die Anreicherung von Stickstoff in diesem Kreislauf begrenzt werden.

Für den Trennstrom 8 ist kein hoher Reinheitsgrad an Wasserstoff erforderlich, weswegen auch das obige Auskreisen eines Teils des Reform-Recyclestroms 14 unschädlich ist. Aus diesem Grund kann es auch vorteilhafterweise sein, dass ein stickstoffhaltiger Spülgasstrom der Niederdruckseite der Membranvorrichtung zum Verdünnen von Wasserstoff zugeführt wird. Anders ausgedrückt dient der Spülgasstrom dazu, insbesondere durch Zufuhr von Stickstoff den Partialdruck von Wasserstoff auf der Niederdruckseite der Membranvorrichtung zu verringern. Auf diese Weise wird es möglich, die Membranvorrichtung bei gleichbleibendem Druck der Niederdruckseite und damit des Trennstroms 8 kleiner auszuführen oder bei gleichbleibender Dimensionierung der Membranvorrichtung die Niederdruckseite mit einem höheren Druck des Trennstroms 8 zu betreiben. Auf diese Weise kann ein Gebläse vor der Zuführung des Trennstroms 8 zu der befeuerten Heizvorrichtung 16 auch dann vermieden werden auch wenn die Heizvorrichtung 16 einen höheren Druck des Trennstroms 8 verlangt. Grundsätzlich kann dieser stickstoffhaltige Spülgasstrom einer beliebigen Quelle entstammen. Bevorzugt ist jedoch speziell, dass der stickstoffhaltige Spülgasstrom aus dem Stickstoffstrom 13 gewonnen wird.

Auch für den CO2-Entferner 4 ist grundsätzlich ein beliebiger Aufbau und eine im Grunde beliebige Funktionsweise denkbar. Eine bevorzugte Ausführungsform sieht vor, dass der CO2-Entferner 4 eine Waschanordnung 26 zum Auswaschen von Kohlenstoffdioxid aus dem Methanol-Restgasstrom 3 aufweist. Mit der Waschanordnung 26 kann also wirksam das Kohlenstoffdioxid aus dem Teil des Methanol-Restgasstroms 3 entfernt werden, welcher dem CO2-Entferner 4 zugeführt wird. Ebenso vorzugsweise weist der CO2-Entferner 4 eine Kompressoranordnung 27 zur Druckerhöhung des ausgewaschenen Kohlenstoffdioxids und zum Erhalten des CO2-Produktstroms 6 auf. Diese Kompressoranordnung 27 erlaubt es dann, den CO2-Produktstrom 6 mit einem für die Einlagerung ausreichenden Druck bereitzustellen. Vorzugsweise weist der CO2-Produktstrom 6, insbesondere nach Druckerhöhung durch die Kompressoranordnung 27, einen Druck von mindestens 90 bar und besonders bevorzugt von mindestens 100 bar auf. Neben der bereits erwähnten Einlagerung des CO2-Produktstroms 6 sieht eine weitere bevorzugte Variante die Möglichkeit vor, dass der CO2-Produktstrom 6 für die Herstellung von Harnstoff (Urea) verwendet wird.

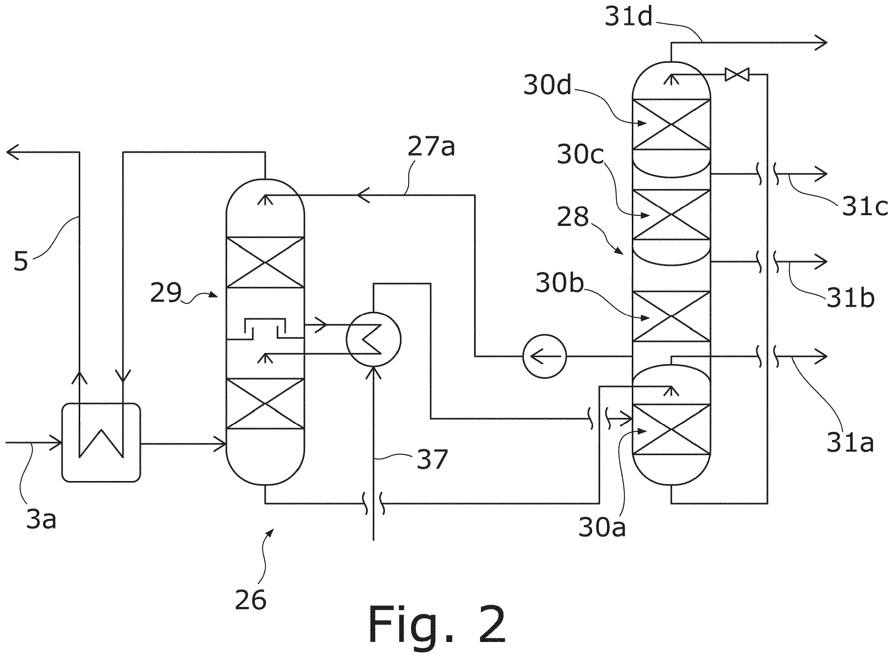

Für die Funktionsweise der Waschanordnung 26 sind unterschiedliche Ansätze möglich. Es kann sein, dass die Waschanordnung 26 das Kohlenstoffdioxid durch eine chemische Wäsche aus dem Methanol-Restgasstrom 3 auswäscht. Bei einer solchen chemischen Wäsche kann das Waschmedium beispielsweise Ammoniak aufweisen oder aus Ammoniak bestehen. Auch kann es sich um eines der bekannten aminbasierten Wascherfahren handeln wie beispielsweise Oasis, aMDEA, MDEA, MEA, DEA, KS1, Econamine. Eine andere, bevorzugte Variante sieht vor, dass die Waschanordnung 26 das Kohlenstoffdioxid durch eine physikalische Wäsche aus dem Methanol-Restgasstrom 3 auswäscht. Im gezeigten Ausführungsbeispiel wäscht die Waschanordnung 26 das Kohlenstoffdioxid speziell aus dem ersten Teilstrom 3a des Methanol-Restgasstroms 3. Beispielsweise kann es sich bei dem eingesetzten physikalischen Waschverfahren um die bekannten Verfahren Rectisol, Purisol, Selexol oder Sulfinol handeln. Bezüglich der Waschanordnung 26 ist bevorzugt, dass sie einen kalten Kreislauf 27a mit einem Waschmedium und eine Regenerationsvorrichtung 28 aufweist. Vorzugsweise weist das Waschmedium Methanol auf. Die Fig. 2 bietet eine entsprechende Darstellung. Aus dieser Darstellung geht weiter hervor, dass die Waschanordnung 26 bevorzugt eine Absorptionsvorrichtung 29 zur Aufnahme des Kohlenstoffdioxids in das Waschmedium aufweist.

Vorteilhafterweise ist die Regenerationsvorrichtung 28 zur Abgabe von Kohlenstoffdioxid aus dem Waschmedium eingerichtet. Grundsätzlich kann diese Regenerationsvorrichtung 28 beliebig ausgestaltet sein. Einerseits kann das Waschmedium in der Regenerationsvorrichtung 28 zur Abgabe des Waschmediums erhitzt werden. Gemäß der Darstellung in der Fig. 2 kann es aber auch sein, dass die Regenerationsvorrichtung 28 eine Vielzahl von Entspannungsstufen 30a-d aufweist, sodass die Regenerationsvorrichtung 28 eine Vielzahl von CO2-Teilströmen 31a-d mit Kohlenstoffdioxid abgibt. Da in den verschiedenen Entspannungsstufen regelmäßig das Waschmedium auf einen jeweils unterschiedlichen Druck entspannt wird, ist bevorzugt vorgesehen, dass die Vielzahl von CO2-Teilströmen31a-d mit einem jeweils unterschiedlichen Druck abgegeben wird.

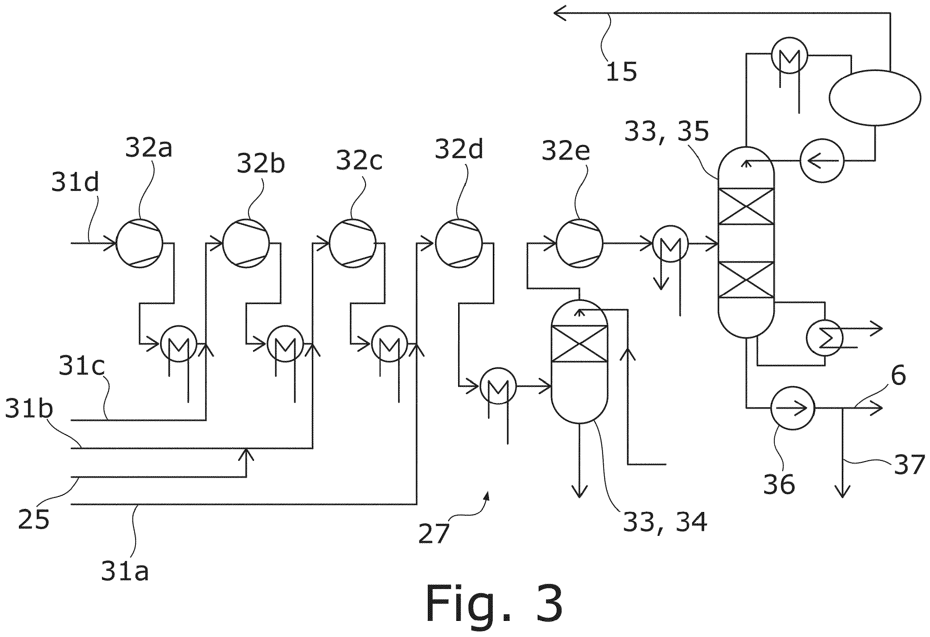

Hinsichtlich der Kompressoranordnung 27 des CO2-Entferners 4 ist bevorzugt, dass - wie in der Fig. 3 dargestellt - die Kompressoranordnung 27 eine Vielzahl von prozesstechnisch hintereinander geschalteten Kompressorstufen 32a-e aufweist. Mit anderen Worten erhöht jede Kompressorstufe 32a-e - bis auf die prozesstechnisch zuerst geschaltete Kompressorstufe 32a - den Druck des Stroms, welcher von der jeweils vorgeschalteten Kompressorstufe 32a-e bereitgestellt wird. Bezogen auf den Strom, welcher von der prozesstechnisch zuerst geschalteten Kompressorstufe 32a aufgenommenen wird, summiert sich also die Druckerhöhung der einzelnen Kompressorstufen 32a-e zu einer Gesamtdruckerhöhung. Bereits nach der ersten Kompressorstufe 32a wird auf diese Weise der CO2-Produktstrom 6 erhalten, welchem dann weitere Ströme zugeführt werden können, wie untenstehend noch erläutert wird.

Verflüssigtes Kohlenstoffdioxid oder Kohlenstoffdioxid, das sich im überkritischen Zustand befindet, ist für die Weiterverarbeitung und den Transport besonders geeignet. Ein Stoff befindet sich im überkritischen Zustand, wenn die Temperatur und der Druck so weit erhöht wird, dass sich die Dichten von flüssiger Phase und Gasphase angleichen. Der Unterschied zwischen diesen beiden Aggregatszuständen verschwindet dann. Für Kohlenstoffdioxid wird der überkritische Zustand bereits bei einer Temperatur von 31° C und einem Druck von 73,8 bar erreicht. Es kann daher sein, dass die Kompressoranordnung 27 zur Druckerhöhung bis zu einer Verflüssigung des CO2-Produktstroms 6 eingerichtet ist. Besonders bevorzugt ist es jedoch, dass die Kompressoranordnung 27 zur Druckerhöhung bis zum Erreichen eines überkritischen Zustands des CO2-Produktstroms 6 eingerichtet ist. In diesem Fall liegt die Temperatur des CO2-Produktstroms 6 oberhalb der kritischen Temperatur und der Druck des CO2-Produktstroms 6 oberhalb des kritischen Drucks.

Neben den Kompressorstufen 32a-e kann die Kompressoranordnung 27 auch Vorrichtungen zur Reinigung des CO2-Produktstroms 6 aufweisen. So ist bevorzugt, dass die Kompressoranordnung 27 eine zumindest teilweise den Kompressorstufen 32a-e prozesstechnisch nachgelagerte Reinigungsanordnung 33 zum Entfernen von Methanol und zum Erhalten des weiteren Reform-Recyclestroms 15 aufweist.

Diese Reinigungsanordnung 33 weist bevorzugt eine Wasserwäsche 34 zur Reinigung des CO2-Produktstroms 6 mit Wasser auf. Eine solche Wäsche mit Wasser ist geeignet, ggf. verbliebenes Methanol zu entfernen. Ebenso kann die Kompressoranordnung 27 auch eine CO2-Destillation 35 aufweisen, wobei speziell der weitere Reform-Recyclestrom 15 aus der CO2-Destillation 35 erhalten werden kann. Durch die CO2-Destillation 35 kann insbesondere ggf. in dem CO2-Produktstrom 6 verbliebenes Methan, Kohlenstoffmonoxid oder Wasserstoff abgetrennt werden und zur weiteren Verwertung zurückgeführt werden. Wie in der Fig. 3 dargestellt, kann die Reinigungsanordnung 33 prozesstechnisch den Kompressorstufen 32a-e zwischengeschaltet angeordnet sein. Die CO2-Destillation 35 kann dann der Reinigungsanordnung 33 prozesstechnisch nachgelagert sein. Auf diese Weise kann die CO2-Destillation 35 bei einem Druck betrieben werden, der höher ist als der Druck in der Reinigungsanordnung 33.

Alternativ oder zusätzlich zu der Reinigungsanordnung 33 kann die Kompressoranordnung 27 eine Flüssigkeitspumpe 36 zum Verpumpen des CO2-Produktstroms 6 aufweisen. Die weitere Druckerhöhung einer Flüssigkeit oder eines Stoffes im überkritischen Zustand durch eine solche Flüssigkeitspumpe 36 ist dabei effizienter möglich als bei einem gasförmigen Stoff. Vorteilhafterweise wird ferner ein insbesondere flüssiger Teilstrom des CO2-Produktstroms 6 für die Kühlung des kalten Kreislaufs 27a verwendet. Speziell kann eine Kühlung des kalten Kreislaufs 27a durch das Verdampfen eines Kohlenstoffdioxidstroms 37 erfolgen, wobei vorzugsweise der Kohlenstoffdioxidstroms 37 vom CO2-Produktstrom 6 abgezweigt wird. Nach Kühlung des kalten Kreislaufs 27a kann der Kohlenstoffdioxidstroms 37 der Regenerationsvorrichtung 28 zugeführt werden. Auf diese Weise tritt durch das Abzweigen kein Verlust an Kohlenstoffdioxid ein. Bei dem Kohlenstoffdioxidstrom 37 handelt es sich vorzugsweise um einen flüssigen Kohlenstoffdioxidstrom 37 oder um einen Kohlenstoffdioxidstrom 37 im überkritischen Zustand.

Das Vorsehen von prozesstechnisch hintereinander geschalteten Kompressorstufen 32a-e hat insbesondere den Vorteil, dass auch Ströme mit untereinander verschiedenem Druck besser zusammengeführt werden können. So ist bevorzugt vorgesehen, dass der Kompressoranordnung 27 mehrere Teilströme von ausgewaschenem Kohlenstoffdioxid zwischen jeweils unterschiedlichen Kompressorstufen 32a-e der Vielzahl zur Druckerhöhung zugeführt werden. Auf diese Weise müssen alle Teilströme mit einem höheren Druck nur noch von nachgelagerten Kompressorstufen 32a-e verarbeitet werden. Im Ergebnis können die ersten Kompressorstufen 32a-e kleiner dimensioniert werden. Bezogen auf die oben beschriebene mehrstufige Regenerationsvorrichtung 28 der Waschanordnung 26 ist es daher bevorzugt, dass die Vielzahl von CO2-Teilströmen31a-d zwischen jeweils unterschiedlichen Kompressorstufen 32a-e der Vielzahl zur Druckerhöhung zugeführt wird. Dieser Sachverhalt ist insbesondere in der Fig. 3 gezeigt.

Neben den CO2-Teilströmen31a-d aus der Regenerationsvorrichtung 28 können aber auch weitere Ströme der Kompressoranordnung 27 zum Erhalten des CO2-Produktstroms 6 zugeführt werden. So ist es bevorzugt, dass der Entspannungs-Restgasstrom 25 - welcher ja aus dem Entspannungs-Tank 24 erhalten wurde - zwischen zwei Kompressorstufen 32a-e der Kompressoranordnung 27 zugeführt wird. Durch seine höhere Reinheit kann es sein, dass er nicht durch die Waschanordnung 26 behandelt werden muss.

Für die oben beschriebene Variante mit der Shiftkonvertierung 38 und dem weiteren CO2-Entferner ist es bevorzugt, dass auch der weitere CO2-Produktstrom der Kompressoranordnung 27 zwischen zwei Kompressorstufen 32a-e der Kompressoranordnung 27 zum Erhalten des CO2-Produktstroms 6 zugeführt wird, da der weitere CO2-Produktstrom bereits einen vergleichsweise hohen Druck aufweist.

## Patentansprüche

1. Verfahren zur Synthese von Methanol, wobei ein Synthesegasstrom (1) mit Wasserstoff und Kohlenstoffoxiden einer Methanol-Reaktoranordnung (2) zur teilweisen Umwandlung in Methanol zugeführt wird, wobei aus der Methanol-Reaktoranordnung (2) ein Methanol-Restgasstrom (3) erhalten wird, welcher Methanol-Restgasstrom (3) zumindest teilweise einem CO2-Entferner (4) zugeführt wird, aus welchem ein Synthese-Recyclestrom (5) und ein CO2-Produktstrom (6) erhalten wird, welcher CO2-Produktstrom (6) einen höheren molaren Kohlenstoffdioxidanteil aufweist als der Methanol-Restgasstrom (3) und wobei der Synthese-Recyclestrom (5) teilweise der Methanol-Reaktoranordnung (2) zugeführt wird, **dadurch gekennzeichnet, dass** der Synthese-Recyclestrom (5) teilweise einem Wasserstofftrenner (7) zugeführt wird, aus welchem ein Trennstrom (8) erhalten wird, welcher einen höheren molaren Wasserstoffanteil aufweist als der Synthese-Recyclestrom (5).

2. Verfahren nach Anspruch 1, **dadurch gekennzeichnet, dass** der Synthesegasstrom (1) in einer Synthesegasreaktoranordnung (9) aus einem kohlenstoffhaltigen Energieträgerstrom (10) gewonnen wird, vorzugsweise, dass zum Gewinnen des Synthesegasstroms (1) ein sauerstoffhaltiger Strom (11) der Synthesegasreaktoranordnung (9) zugeführt wird.

3. Verfahren nach Anspruch 1 oder 2, **dadurch gekennzeichnet, dass** in der Synthesegasreaktoranordnung (9) der Synthesegasstrom (1) durch eine autotherme Reformierung aus dem kohlenstoffhaltigen Energieträgerstrom (10) gewonnen wird, vorzugsweise, dass der sauerstoffhaltige Strom (11) aus einer Luftzerlegungsvorrichtung (12) zum Gewinnen eines Stickstoffstroms (13) gewonnen wird, insbesondere, dass der sauerstoffhaltige Strom (11) im Wesentlichen aus Sauerstoff besteht.

4. Verfahren nach einem der Ansprüche 1 bis 3, **dadurch gekennzeichnet, dass** aus dem Wasserstofftrenner (7) ein Reform-Recyclestrom (14) erhalten wird, welcher einen höheren molaren Methananteil aufweist als der Synthese-Recyclestrom (5), insbesondere, dass der Reform-Recyclestrom (14) der Synthesegasreaktoranordnung (9) zum Gewinnen des Synthesegasstroms (1) zugeführt wird.

5. Verfahren nach einem der Ansprüche 1 bis 4, **dadurch gekennzeichnet, dass** aus dem CO2-Entferner (4) ein weiterer Reform-Recyclestrom (15) erhalten wird, vorzugsweise, dass der weitere Reform-Recyclestrom (15) mit dem Reform-Recyclestrom (14) zusammengeführt wird, insbesondere, dass der weitere Reform-Recyclestrom (15) der Synthesegasreaktoranordnung (9) zum Gewinnen des Synthesegasstroms (1) zugeführt wird, weiter insbesondere, dass der weitere Reform-Recyclestrom (15) einen höheren molaren Methananteil aufweist als der Methanol-Restgasstrom (3), weiter vorzugsweise, dass der Trennstrom (8) einer befeuerten Heizeinrichtung (16) zur Verbrennung zugeführt wird.

6. Verfahren nach einem der Ansprüche 1 bis 5, **dadurch gekennzeichnet, dass** der Synthesegasstrom (1) vor Zuführung zu der Methanol-Reaktoranordnung (2) zumindest teilweise einer Shiftkonvertierung (38) für eine Wassergas-Shift-Reaktion zugeführt wird, vorzugsweise, sodass ein molarer Anteil an Wasserstoff im Synthesegasstrom (1) erhöht wird.

7. Verfahren nach einem der Ansprüche 1 bis 6, **dadurch gekennzeichnet, dass** die Methanol-Reaktoranordnung (2) eine Methanol-Trennvorrichtung (18) zum Gewinnen des Methanol-Restgasstroms (3) und eines Rohmethanolstroms (19) aus einem Reaktor-Produktstrom (20) umfasst, vorzugsweise, dass die Methanol-Trennvorrichtung (18) eine Kondensationsvorrichtung (23) zum Gewinnen des Rohmethanolstroms (19) und des Methanol-Restgasstroms (3) aus dem Reaktor-Produktstrom (20) durch Kondensation umfasst, insbesondere, dass die Methanol-Trennvorrichtung (18) einen Entspannungs-Tank (24) zum Gewinnen eines Entspannungs-Restgasstroms (25) aus dem Reaktor-Produktstrom (20) und/oder aus dem Rohmethanolstrom (19) umfasst, weiter insbesondere, dass der Entspannungs-Restgasstrom (25) dem CO2-Entferner (4) zugeführt wird.

8. Verfahren nach einem der Ansprüche 1 bis 7, **dadurch gekennzeichnet, dass** der Wasserstofftrenner (7) eine Membranvorrichtung zum Abtrennen von Wasserstoff aufweist, vorzugsweise, dass der Trennstrom (8) aus einer Niederdruckseite der Membranvorrichtung und der Reform-Recyclestrom (14) aus einer Hochdruckseite der Membranvorrichtung erhalten wird, insbesondere, dass der Trennstrom (8) mit einem niedrigeren Druck als der Reform-Recyclestrom (14) aus der Membranvorrichtung erhalten wird.

9. Verfahren nach Anspruch 8, **dadurch gekennzeichnet, dass** ein stickstoffhaltiger Spülgasstrom der Niederdruckseite der Membranvorrichtung zum Verdünnen von Wasserstoff, zugeführt wird, insbesondere, dass der stickstoffhaltige Spülgasstrom aus dem Stickstoffstrom (13) gewonnen wird.

10. Verfahren nach einem der Ansprüche 1 bis 9, **dadurch gekennzeichnet, dass** der CO2-Entferner (4) eine Waschanordnung (26) zum Auswaschen von Kohlenstoffdioxid aus dem Methanol-Restgasstrom (3) aufweist, vorzugsweise, dass der CO2-Entferner (4) eine Kompressoranordnung (27) zur Druckerhöhung des ausgewaschenen Kohlenstoffdioxids und zum Erhalten des CO2-Produktstroms (6) aufweist.

11. Verfahren nach Anspruch 10, **dadurch gekennzeichnet, dass** die Waschanordnung (26) das Kohlenstoffdioxid durch eine chemische Wäsche und/oder durch eine physikalische Wäsche aus dem Methanol-Restgasstrom (3) auswäscht, vorzugsweise, dass die Waschanordnung (26) einen kalten Kreislauf (27a) mit einem Waschmedium mit Methanol und einer Regenerationsvorrichtung (28) aufweist.

12. Verfahren nach Anspruch 10 oder 11, **dadurch gekennzeichnet, dass** die Regenerationsvorrichtung (28) zur Abgabe von Kohlenstoffdioxid aus dem Waschmedium eingerichtet ist, vorzugsweise, dass die Regenerationsvorrichtung (28) eine Vielzahl von Entspannungsstufen (30a-d) aufweist, sodass die Regenerationsvorrichtung (28) eine Vielzahl von CO2-Teilströmen (31a-d) mit Kohlenstoffdioxid abgibt, insbesondere dass die Vielzahl von CO2-Teilströmen(31a-d) mit einem jeweils unterschiedlichen Druck abgegeben wird.

13. Verfahren nach einem der Ansprüche 10 bis 12, **dadurch gekennzeichnet, dass** die Kompressoranordnung (27) eine Vielzahl von prozesstechnisch hintereinander geschalteten Kompressorstufen (32a-e) aufweist, insbesondere, dass die Kompressoranordnung (27) zur Druckerhöhung bis zu einer Verflüssigung des CO2-Produktstroms (6) eingerichtet ist, insbesondere, dass die Kompressoranordnung (27) eine den Kompressorstufen (32a-e) zumindest teilweise prozesstechnisch nachgelagerte Reinigungsanordnung (33) zum Entfernen von Methanol und zum Erhalten des weiteren Reform-Recyclestroms (15) und/oder eine Flüssigkeitspumpe (36) zum Verpumpen des CO2-Produktstroms (6) aufweist.

14. Verfahren nach Anspruch 13, **dadurch gekennzeichnet, dass** der Kompressoranordnung (27) mehrere Teilströme von ausgewaschenem Kohlenstoffdioxid zwischen jeweils unterschiedlichen Kompressorstufen (32a-e) der Vielzahl zur Druckerhöhung zugeführt werden, vorzugsweise, dass die Vielzahl von CO2-Teilströmen(31a-d) zwischen jeweils unterschiedlichen Kompressorstufen (32a-e) der Vielzahl zur Druckerhöhung zugeführt wird, insbesondere, dass der Entspannungs-Restgasstrom (25) zwischen zwei Kompressorstufen (32a-e) der Kompressoranordnung (27) zugeführt wird.

15. Anlage zur Synthese von Methanol mit einer Methanol-Reaktoranordnung (2), welcher ein Synthesegasstrom (1) mit Wasserstoff und Kohlenstoffoxiden zur teilweisen Umwandlung in Methanol und zum Erhalten eines Methanol-Restgasstroms (3) zugeführt wird, einem CO2-Entferner (4), welchem der Methanol-Restgasstrom (3) zumindest teilweise zum Erhalten eines Synthese-Recyclestroms (5) und eines CO2-Produktstroms (6) zugeführt wird, welcher CO2-Produktstrom (6) einen höheren molaren Kohlenstoffdioxidanteil aufweist als der Methanol-Restgasstrom (3) und wobei der Synthese-Recyclestrom (5) teilweise der Methanol-Reaktoranordnung (2) zugeführt wird, **dadurch gekennzeichnet, dass** die Anlage einen Wasserstofftrenner (7) aufweist, welchem zum Erhalten eines Trennstroms (8) der Synthese-Recyclestrom (5) teilweise zugeführt wird und dass der Trennstrom (8) einen höheren molaren Wasserstoffanteil aufweist als der Synthese-Recyclestrom (5).

## Claims

1. A method for synthesizing methanol, a synthesis gas stream (1) comprising hydrogen and carbon oxides being fed to a methanol reactor arrangement (2) for partial conversion into methanol, a methanol residual gas stream (3) being obtained from the methanol reactor arrangement (2), at least part of said methanol residual gas stream (3) being fed to a CO2 remover (4), from which a synthesis recycle stream (5) and a CO2 product stream (6) are obtained, said CO2 product stream (6) having a higher molar carbon dioxide content than the methanol residual gas stream (3) and part of the synthesis recycle stream (5) being fed to the methanol reactor arrangement (2), **characterized in that** part of the synthesis recycle stream (5) is fed to a hydrogen separator (7), from which a separation stream (8) is obtained, said separation stream (8) having a higher molar hydrogen content than the synthesis recycle stream (5).

2. The method according to claim 1, **characterized in that** the synthesis gas stream (1) is extracted from a carbon-containing energy carrier stream (10) in a synthesis gas reactor arrangement (9), preferably that an oxygen-containing stream (11) is fed to the synthesis gas reactor arrangement (9) for extracting the synthesis gas stream (1).

3. The method according to claim 1 or 2, **characterized in that** in the synthesis gas reactor arrangement (9), the synthesis gas stream (1) is extracted from the carbon-containing energy carrier stream (10) by autothermal reforming, preferably that the oxygen-containing stream (11) is extracted from an air separation device (12) for extracting a nitrogen stream (13), particularly that the oxygen-containing stream (11) consists essentially of oxygen.

4. The method according to any one of claims 1 to 3, **characterized in that** a reform recycle stream (14) is obtained from the hydrogen separator (7), said reform recycle stream (14) having a higher molar methane content than the synthesis recycle stream (5), particularly that the reform recycle stream (14) is fed to the synthesis gas reactor arrangement (9) for extracting the synthesis gas stream (1).

5. The method according to any one of claims 1 to 4, **characterized in that** a further reform recycle stream (15) is obtained from the CO2 remover (4), preferably that the further reform recycle stream (15) is combined with the reform recycle stream (14), particularly that the further reform recycle stream (15) is fed to the synthesis gas reactor arrangement (9) for extracting the synthesis gas stream (1), further particularly that the further reform recycle stream (15) has a higher molar methane content than the methanol residual gas stream (3), further preferably that the separation stream (8) is fed to a fired heating device (16) for combustion.

6. The method according to any one of claims 1 to 5, **characterized in that** at least part of the synthesis gas stream (1) is fed to a shift conversion (38) for a water-gas shift reaction before being fed to the methanol reactor arrangement (2), preferably so that a molar content of hydrogen is increased in the synthesis gas stream (1).

7. The method according to any one of claims 1 to 6, **characterized in that** the methanol reactor arrangement (2) comprises a methanol separation device (18) for extracting the methanol residual gas stream (3) and a crude methanol stream (19) from a reactor product stream (20), preferably that the methanol separation device (18) comprises a condensation device (23) for extracting the crude methanol stream (19) and the methanol residual gas stream (3) from the reactor product stream (20) by condensation, particularly that the methanol separation device (18) comprises an expansion tank (24) for extracting an expansion residual gas stream (25) from the reactor product stream (20) and/or from the crude methanol stream (19), further particularly that the expansion residual gas stream (25) is fed to the CO2 remover (4).

8. The method according to any one of claims 1 to 7, **characterized in that** the hydrogen separator (7) comprises a membrane device for separating hydrogen, preferably that the separation stream (8) is obtained from a low-pressure side of the membrane device and the reform recycle stream (14) is obtained from a highpressure side the membrane device, particularly that the separation stream (8) is obtained from the membrane device at a lower pressure than the reform recycle stream (14).

9. The method according to claim 8, **characterized in that** a nitrogen-containing purge gas stream is fed to the low-pressure side of the membrane device for diluting hydrogen, particularly that the nitrogen-containing purge gas stream is extracted from the nitrogen stream (13) .

10. The method according to any one of claims 1 to 9, **characterized in that** the CO2 remover (4) comprises a scrubbing arrangement (26) for scrubbing carbon dioxide from the methanol residual gas stream (3), preferably that the CO2 remover (4) comprises a compressor arrangement (27) for increasing the pressure of the scrubbed carbon dioxide and for obtaining the CO2 product stream (6).

11. The method according to claim 10, **characterized in that** the scrubbing arrangement (26) scrubs the carbon dioxide out of the methanol residual gas stream (3) by chemical scrubbing and/or by physical scrubbing, preferably that the scrubbing arrangement (26) comprises a cold circuit (27a) having a scrubbing medium with methanol and a regeneration device (28).

12. The method according to claim 10 or 11, **characterized in that** the regeneration device (28) is configured to yield carbon dioxide from the scrubbing medium, preferably that the regeneration device (28) comprises a plurality of expansion stages (30a-d) , so that the regeneration device (28) delivers a plurality of CO2 partial streams (31a-d) with carbon dioxide, particularly that the large number of CO2 partial streams (31a-d) is delivered at a different pressure in each case.

13. The method according to any one of claims 10 to 12, **characterized in that** the compressor arrangement (27) comprises a plurality of compressor stages (32a-e) connected in series in terms of process technology, particularly that the compressor arrangement (27) is configured to increase the pressure until the CO2 product stream (6) is liquefied, particularly that the compressor arrangement (27) comprises a cleaning arrangement (33), part of which is downstream of the compressor stages (32a-e) in terms of process technology, for removing methanol and for obtaining the further reform recycle stream (15) and/or a liquid pump (36) for pumping the CO2 product stream (6).

14. The method according to claim 13, **characterized in that** the compressor arrangement (27) is fed with a plurality of partial streams of scrubbed carbon dioxide between respectively different compressor stages (32a-e) of the plurality for increasing the pressure, preferably that the plurality of CO2 partial streams (31a-d) is fed to the plurality between respectively different compressor stages (32a-e) for increasing the pressure, particularly that the expansion residual gas stream (25) is fed to the compressor arrangement (27) between two compressor stages (32a-e) .

15. A system for synthesizing methanol comprising a methanol reactor arrangement (2), to which is fed a synthesis gas stream (1) comprising hydrogen and carbon oxides for partial conversion into methanol and for obtaining a methanol residual gas stream (3), a CO2 remover (4), to which at least part of the methanol residual gas stream (3) is fed for obtaining a synthesis recycle stream (5) and a CO2 product stream (6), said CO2 product stream (6) having a higher molar carbon dioxide content than the methanol residual gas stream (3) and part of the synthesis recycle stream (5) being fed to the methanol reactor arrangement (2), **characterized in that** the system comprises a hydrogen separator (7) to which at least part of the synthesis recycle stream (5) is fed to obtain a separating stream (8) and that the separating stream (8) has a higher molar hydrogen content than the synthesis recycle stream (5).

## Revendications

1. Procédé de synthèse de méthanol, consistant à introduire un flux de gaz de synthèse (1), avec de l'hydrogène et des oxydes de carbone, dans un système de réacteur de méthanol (2), afin qu'il soit partiellement transformé en méthanol, un flux gazeux résiduel de méthanol (3) étant obtenu à l'issue du système de réacteur de méthanol (2), le flux gazeux résiduel de méthanol (3) étant au moins partiellement introduit dans un dispositif d'élimination de CO2 (4) à l'issue duquel on obtient un flux de synthèse à recycler (5) et un flux de produit de CO2 (6), le flux de produit de CO2 (6) ayant une proportion molaire de dioxyde de carbone plus élevée que le flux résiduel gazeux de méthanol (3), et le flux de synthèse à recycler (5) étant partiellement recyclé dans le système de réacteur de méthanol (2), **caractérisé en ce que** le flux de synthèse à recycler (5) est partiellement introduit dans un dispositif de séparation d'hydrogène (7) l'issue duquel on obtient un flux séparé (8) ayant une proportion molaire d'hydrogène plus élevée que le flux de synthèse à recycler (5).

2. Procédé selon la revendication 1, **caractérisé en ce que** le flux de gaz de synthèse (1) est obtenu, à partir d'un flux de vecteur d'énergie (10) contenant du carbone, dans un système de réacteur de gaz de synthèse (9), le système de réacteur de gaz de synthèse (9) étant de préférence alimenté en un flux contenant de l'oxygène (11) afin d'obtenir le flux de gaz de synthèse (1).

3. Procédé selon les revendications 1 ou 2, **caractérisé en ce qu'**au sein du système de réacteur de gaz de synthèse (9), le flux de gaz de synthèse (1) est obtenu par reformage autotherme à partir du flux de vecteur d'énergie (10) contenant du carbone, le flux contenant de l'oxygène (11) étant de préférence issu d'une installation de liquéfaction fractionnée d'air (12) destinée à obtenir un flux d'azote (13), le flux contenant de l'oxygène (11) étant notamment constitué sensiblement d'oxygène.

4. Procédé selon l'une des revendications 1 à 3, **caractérisé en ce qu'**à partir du dispositif de séparation d'hydrogène (7), on obtient un flux de reformage à recycler (14) qui a une proportion molaire de méthane plus élevée que le flux de synthèse à recycler (5), le flux de reformage à recycler (14) étant notamment introduit dans le système de réacteur de gaz de synthèse (9) afin d'obtenir le flux de gaz de synthèse (1).

5. Procédé selon l'une des revendications 1 à 4, **caractérisé en ce qu'**à l'issue du dispositif d'élimination de CO2 (4), on obtient un flux de reformage à recycler supplémentaire (15), le flux de reformage à recycler supplémentaire (15) étant de préférence réuni avec le flux de reformage à recycler (14), le flux de reformage à recycler supplémentaire (15) étant notamment introduit dans le système de réacteur de gaz de synthèse (9) afin d'obtenir le flux de gaz de synthèse (1), et le flux de reformage à recycler supplémentaire (15) ayant en outre notamment une proportion molaire de méthane plus élevée que le flux gazeux résiduel de méthanol (3), et le flux séparé (8) étant de préférence introduit dans un dispositif de chauffage (16) à combustion afin d'y être brûlé.

6. Procédé selon l'une des revendications 1 à 5, **caractérisé en ce qu'**avant d'être introduit dans le système de réacteur de méthanol (2), le flux de gaz de synthèse (1) est au moins partiellement soumis à une conversion de décalage d'équilibre (38) en vue d'une réaction du gaz à l'eau, faisant préférentiellement en sorte que la proportion molaire d'hydrogène dans le flux de gaz de synthèse (1) augmente.

7. Procédé selon l'une des revendications 1 à 6, **caractérisé en ce que** le système de réacteur de méthanol (2) comprend une installation de séparation de méthanol (18) permettant d'obtenir, à partir d'un flux de produit de réacteur (20), le flux gazeux résiduel de méthanol (3) et un flux de méthanol brut (19), l'installation de séparation de méthanol (18) comprenant de préférence une installation de condensation (23) permettant d'obtenir, à partir du flux de produit de réacteur (20), le flux de méthanol brut (19) et le flux gazeux résiduel de méthanol (3) par condensation, l'installation de séparation de méthanol (18) comprenant notamment une cuve de détente (24) permettant d'obtenir, à partir du flux de produit de réacteur (20) et/ou à partir du flux de méthanol brut (19), un flux gazeux résiduel de détente (25), le flux gazeux résiduel de détente (25) étant en outre notamment introduit dans le dispositif d'élimination de CO2 (4).

8. Procédé selon l'une des revendications 1 à 7, **caractérisé en ce que** le dispositif de séparation d'hydrogène (7) comporte un organe à membrane permettant de séparer l'hydrogène, le flux séparé (8) étant de préférence obtenu depuis un côté basse pression dudit organe à membrane et le flux de reformage à recycler (14) étant de préférence obtenu depuis un côté haute pression dudit organe à membrane, le flux séparé (8) étant notamment obtenu de manière à ce qu'à l'issue dudit organe à membrane, il présente une pression moins élevée que le flux de reformage à recycler (14).

9. Procédé selon la revendication 8, **caractérisé en ce que** le côté basse pression dudit organe à membrane est alimenté en un flux de gaz de balayage contenant de l'azote, ledit flux de gaz de balayage contenant de l'azote étant notamment obtenu à partir du flux d'azote (13) .

10. Procédé selon l'une des revendications 1 à 9, **caractérisé en ce que** le dispositif d'élimination de CO2 (4) comporte un système de lavage (26) permettant d'extraire le dioxyde de carbone du flux gazeux résiduel de méthanol (3) par lavage, le dispositif d'élimination de CO2 (4) comportant un système de compression (27) permettant d'augmenter la pression du dioxyde de carbone extrait par lavage et d'obtenir le flux de produit de CO2 (6).

11. Procédé selon la revendication 10, **caractérisé en ce que** le système de lavage (26) extrait le dioxyde de carbone du flux gazeux résiduel de méthanol (3) en mettant en œuvre un lavage chimique et/ou un lavage physique, le système de lavage (26) étant de préférence pourvu d'un circuit fermé froid (27a) comportant un milieu de lavage comprenant du méthanol et d'une installation de régénération (28).

12. Procédé selon les revendications 10 ou 11, **caractérisé en ce que** l'installation de régénération (28) est réalisée de manière à ce qu'elle libère du dioxyde de carbone à partir dudit milieu de lavage, l'installation de régénération (28) comportant de préférence une pluralité d'étages de détente (30a-d) , faisant en sorte que l'installation de régénération (28) libère une pluralité de flux partiels de CO2 (31a-d) comportant du dioxyde de carbone, la pluralité de flux partiels de CO2 (31a-d) étant notamment libérés chacun à une pression différente.

13. Procédé selon l'une des revendications 10 à 12, **caractérisé en ce que** le système de compression (27) comporte une pluralité d'étages de compression (32a-e) disposés les uns en aval des autres au cours du processus, le système de compression (27) étant notamment réalisé de manière à ce qu'il augmente la pression jusqu'à ce que le flux de produit de CO2 (6) soit liquéfié, le système de compression (27) étant notamment pourvu d'un système de nettoyage (33) qui est au moins partiellement disposé en aval des étages de compression (32a-e) au cours du processus et qui est destiné à retirer du méthanol et à obtenir le flux de reformage à recycler supplémentaire (15) et/ou d'une pompe à liquide (36) qui est destinée à évacuer par pompage le flux de produit de CO2 (6).

14. Procédé selon la revendication 13, **caractérisé en ce que** plusieurs flux partiels de dioxyde de carbone extrait par lavage sont introduits, chacun entre différents étages de compression (32a-e) de ladite pluralité visant à augmenter la pression, dans le système de compression (27), la pluralité de flux partiels de CO2 (31a-d) étant de préférence introduite chacun entre différents étages de compression (32a-e) de ladite pluralité visant à augmenter la pression, le flux gazeux résiduel de détente (25) étant notamment introduit entre deux étages de compression (32a-e) du système de compression (27).

15. Installation de synthèse de méthanol pourvue d'un système de réacteur de méthanol (2) dans lequel on introduit un flux de gaz de synthèse (1) comportant de l'hydrogène et des oxydes de carbone, afin de réaliser une conversion partielle en méthanol et d'obtenir un flux gazeux résiduel de méthanol (3), d'un dispositif d'élimination de CO2 (4) dans lequel on introduit au moins partiellement le flux gazeux résiduel de méthanol (3) afin d'obtenir un flux de synthèse à recycler (5) et un flux de produit de CO2 (6), le flux de produit de CO2 (6) ayant une proportion molaire de dioxyde de carbone plus élevée que le flux gazeux résiduel de méthanol (3), et le flux de synthèse à recycler (5) étant partiellement introduit dans un système de réacteur de méthanol (2), **caractérisée en ce que** ladite installation comporte un dispositif de séparation d'hydrogène (7) dans lequel on introduit partiellement le flux de synthèse à recycler (5) afin d'obtenir un flux séparé (8) et que la flux séparé (8) présente une proportion molaire d'hydrogène plus élevée que le flux de synthèse à recycler (5).
